(19) Europäisches Patentamt
European Patent Office
Office européen des brevets

(11) Veröffentlichungsnummer: **0 451 688 B1**

# (12) EUROPÄISCHE PATENTSCHRIFT

(45) Veröffentlichungstag der Patentschrift: **05.10.94**

(51) Int. Cl.$^5$: **A61K 6/033**, A61K 6/027

(21) Anmeldenummer: **91105285.0**

(22) Anmeldetag: **03.04.91**

## (54) Pulverförmige Dental-Einbettmassen mit verbessertem Fliessverhalten.

(30) Priorität: **12.04.90 DE 4011871**

(43) Veröffentlichungstag der Anmeldung:
**16.10.91 Patentblatt 91/42**

(45) Bekanntmachung des Hinweises auf die Patenterteilung:
**05.10.94 Patentblatt 94/40**

(84) Benannte Vertragsstaaten:
**AT BE CH DE DK ES FR GB GR IT LI LU NL SE**

(56) Entgegenhaltungen:
**EP-A- 0 170 020**
**EP-A- 0 217 270**
**GB-A- 2 187 728**

**CHEMICAL ABSTRACTS, vol. 109, no. 20, 14. November 1988, Columbus, Ohio, US;**

**abstract no. 176382J,**

(73) Patentinhaber: **BAYER AG**

**D-51368 Leverkusen (DE)**

(72) Erfinder: **Schwabe, Peter, Dr.**
**Dudweiler Strasse 17**
**W-5090 Leverkusen 1 (DE)**
Erfinder: **Theis, Wilhelm, Dipl.-Ökon.**
**Förstchen 35**
**W-5653 Leichlingen 1 (DE)**
Erfinder: **Voigt, Reiner, Dipl.-Ing.**
**An der Lichtenburg 4**
**W-5090 Leverkusen 1 (DE)**
Erfinder: **Winkel, Jens, Dr.**
**Letterhausstrasse 1**
**W-5000 Köln 71 (DE)**

**EP 0 451 688 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die vorliegende Erfindung betrifft Einbettmassen auf Gips- bzw. Magnesiumoxid/Phosphat-Basis, ihre Herstellung und ihre Verwendung zur Herstellung von Guß-Reparaturmaterialien,bevorzugt im Dentalbereich.

Bei der Herstellung von Inlays, Kronen, Brücken und Modellgußprothesen stellt man mit Hilfe von Abformmaterialien z.B. ein Negativ des interessierenden Bereichs her, das anschließend mit Modellgips ausgegossen wird. Anhand des Gipsmodells werden nun die o. g. Ersatzstücke mit Wachs modelliert. Diese Wachsmodelle, mit einer Eingußleitung versehen, bettet man in eine Masse, bestehend aus einer nachstehend beschriebenen Pulvermischung und einer Anmischflüssigkeit, ein. Durch Erhitzen schmilzt bzw. brennt das Wachs heraus und in den gebildeten Hohlraum wird eine flüssige Metall-Legierung wie Gold- oder Chrom/Kobalt-Legierung eingebracht. Nach dem Erkalten wird die Gußform zerstört und man erhält die o. g. Ersatzstücke, die nach weiterer Bearbeitung wie Polieren, Aufsetzen von Kunststoff-oder Keramikverblendungen die Lücken in der Zahnreihe des Patienten schließen werden.

Einbettmassen zur Herstellung von Gußstücken im Dentalbereich sind seit langem bekannt. Sie bestehen aus einem Feuerfestmaterial wie Quarz und/oder Cristobalit und einem Bindemittel wie Calciumsulfat-Halbhydrat bzw. Magnesiumoxid und Ammoniumdihydrogenphosphat.

Diese handelsüblichen Pulvermischungen werden vor Anwendung mit Anmischflüssigkeiten wie Wasser oder Kieselsol, um eine gebrauchsfähige pastöse Masse zu erhalten, in einem vom Hersteller angegebenen Mischungsverhältnis zu pastösen Massen vermengt und anschließend in die Gußküvette mit dem Wachsmodell eingetragen.

Eine weitere Einbettmassengruppe besteht aus dem pulverförmigen Feuerfestmaterial Quarz/Cristobalit und dem flüssigen Bindemittel Ethysilikat als Anmischflüssigkeit, welche ebenfalls wie oben beschrieben verarbeitet wird.

Typische gipshaltige Einbettmassen enthalten beispielsweise:

45-65 Gew.-% Quarzsand
10-25 Gew.-% Cristobalitmehl
20-35 Gew.-% Calciumsulfat-Hemihydrat
0-5 Gew.-% Verflüssiger (z.B. Melaminharz)
0-2 Gew.-% Beschleuniger (z.B. Kaliumsulfat)
0-2 Gew.-% Verzögerer (z.B. Trinatriumcitrat).

Typische Magnesiumoxid enthaltende Einbettmassen können beispielsweise wie folgt zusammengesetzt sein:

40-50 Gew.-% Quarzsand
10-20 Gew.-% Quarzmehl
15-25 Gew.-% Cristobalitmehl
6-15 Gew.-% Magnesiumoxid
6-15 Gew.-% Ammoniumdihydrogenphosphat.

Die Einbettmassen mit ihren Eigenschaften und Anwendungsmöglichkeiten sind vielfach beschrieben, so in Karl Eichner, Zahnärztliche Werkstoffe und ihre Verarbeitung Band 1, 1988, Dr. Alfred Hüthig Verlag GmbH Heidelberg S. 25 - 47.

Aus der DE-A 37 07 853 sind bereits pulverförmige Dentalformzubereitungen für den Metallguß bekannt geworden, die eine geringere Staubbildung aufweisen, die dadurch gekennzeichnet sind, daß sie neben einer Mischung eines löslichen Phosphates mit einem Magnesiumoxid oder Gips-Hemihydrat als Bindemittel und Quarz und/oder Cristoballit als Feuerfestmaterial, einen oder mehrere flüssige hydrophobe Kohlenwasserstoffe, flüssige hydrophobe Fettsäureester und/oder flüssige hydrophobe Fettsäuren eines Dampfdrucks bei 20 °C von 4,19 mbar oder darunter aufweisen und zusätzlich zwingend ein anionisch grenzflächenaktives Mittel enthalten, das als effektiver Bestandteil zur Verbesserung der Eigenschaften vorliegen muß. Die Anwesenheit von oberflächenaktiven Substanzen kann jedoch die Einbettmassen nachteilig beeinflussen.

Beim Mischen einer pulverförmigen Einbettmasse mit der Flüssigkomponente sollte der Anteil der Flüssigkomponente so niedrig wie möglich sein, da ein hoher Anteil die Abbindezeit verlängert, die Druckfestigkeit der ausgehärteten Masse reduziert und es zu Anhaftungen der Einbettmasse an das Dubliergel auf Agar Agar-Basis kommt.

Andererseits muß die angemischte Einbettmasse gut fließfähig sein, damit es keine Fehlstellen durch einbettmassenfreie Zonen gibt.

Es wurden Einbettmassen auf Calciumsulfathalbhydrat bzw. Magnesiumoxid/Ammoniumphosphat-Basis gefunden, die dadurch gekennzeichnet sind, daß sie Iso-Paraffine der Formel

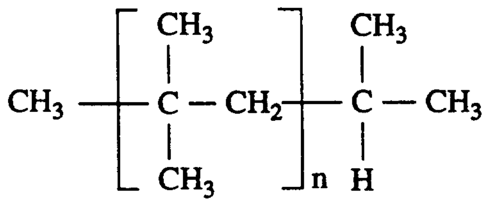

in der

n für 2, 3, 4 oder 5 steht,

enthalten.

Insbesondere werden Iso-Paraffine bevorzugt, bei denen

n für 3 oder 4 steht.

Es ist selbstverständlich möglich, Gemische der Iso-Paraffine einzusetzen. Insbesondere werden 2,2,4,4,6,6,8-Heptamethyl-nonan und 2,2,4,4,6,6,8,8,10-Nonamethyl-undecan bevorzugt.

Die Iso-Paraffine zeigen eine Viskosität im Bereich von 4 bis 8 mPa.s/20 °C und einen Siedepunkt im Bereich von 210 bis 320 °C. Der Flammpunkt liegt im Bereich von 90 bis 130 °C und die Zündtemperatur im Bereich von 375 bis 420 °C.

Vorzugsweise enthalten die erfindungsgemäßen Einbettmassen 0,5 bis 5 Gew.-%, insbesondere 1,5 bis 3 Gew.-% an Iso-Paraffin.

Die erfindungsgemäßen Einbettmassen weisen ein verbessertes Fließverhalten auf. Sie sind außerdem gut durch die Anmischflüssigkeit benetzbar, ohne daß hierzu die Anwesenheit eines grenzflächenaktiven Mittels erforderlich ist.

Im allgemeinen enthalten die erfindungsgemäßen gipsgebundenen Einbettmassen aus Quarz und/oder Cristobalit, Calciumsulfat-Halbhydrat Zuschlagstoffe wie Borsäure, Natriumchlorid, Kaliumchlorid, Trinatriumcitrat, Borax, Kalium-und Natriumsulfat. Die ethylsilikatgebundenen Einbettmassen bestehen im wesentlichen aus Quarzmodifikationen, das Bindemittel wird vor der Verarbeitung als flüssiges Ethylsilikat hinzugegeben. Aus Magnesiumoxid und Ammoniumdihydrogenphosphat als Bindemittel und Quarz und Cristobalit als Feuerfestmaterial bestehen die erfindungsgemäßen phosphatgebundenen Einbettmassen.

Die erfindungsgemäßen Einbettmassen werden hergestellt, indem man dem pulverförmigen Gemisch der oben genannten Komponenten das Iso-Paraffin vorzugsweise bei Raumtemperatur des Mischguts zusetzt. Es ist jedoch auch möglich, nur die eine oder einige der Komponenten mit Iso-Paraffin zu behandeln und dann den restlichen Komponenten zuzugeben. Der Zusatz des Iso-Paraffins erfolgt zweckmäßigerweise in einem Schaufelmischer, z. B. einem Lödige-Mischer, dessen Wand mit Düsen versehen ist, durch welche das Iso-Paraffin auf das pulverförmige Gemisch aufgesprüht wird. Eine gleichmäßige Verteilung des Isoparaffins (Beschichtung der pulverförmigen Einbettmasse) wird hierdurch optimal erreicht.

Abgesehen von dem verbesserten Fließverhalten zeichnen sich die erfindungsgemäßen Einbettmassen überraschenderweise durch ein besseres Benetzen und Dispergieren mit der Anmischflüssigkeit und durch ein günstiges Mischungsverhältnis von Pulver zu Flüssigkeit aus, und die Abbindeexpansion wird reduziert. Es wurde auch eine verringerte Staubentwicklung der erfindungsgemäßen Pulvermischungen beobachtet.

Beispiele

Eine phosphatgebundene Einbettmasse wurde durch Vermischen folgender Komponenten hergestellt:

| | |
|---|---|
| Quarzsand | 45 Gew.-% |
| Quarzmehl | 15 Gew.-% |
| Cristobalitmehl | 20 Gew.-% |
| Magnesiumoxid | 10 Gew.-% |
| Ammoniumdihydrogenphosphat | 9 Gew.-% |
| Farbstoff | 1 Gew.-% |

Das pulverförmige Material wurde in mehrere Portionen geteilt, die in folgender Weise mit verschiedenen Beschichtungsmitteln behandelt wurden: Das Gemisch wurde in einem Pulvermischer (Pflugscharmischer mit 3 l Volumen) vorgelegt und von oben mittels einer Sprühpistole mit dem jeweiligen Beschichtungsmittel besprüht. Nach 10 Minuten wurde der Mischer abgestellt

Die Ermittlung der Meßwerte für Verarbeitungszeit, Abbindezeit und -temperatur, Fließverhalten, Druckfestigkeit und Abbindeexpansion wurde nach DIN 13919 Teil 2 durchgeführt, wobei das Mischungsverhältnis bei 100 g pulverförmiger Einbettmasse zu 14 g Kieselsol lag, um eine gebrauchsfähige pastöse Masse zu erhalten.

Außerdem wurden die so beschichteten Proben auf ihre relative Staubentwicklung geprüft. Das hierzu benutzte Gerät ist eine Kammer mit den Abmessungen 50 x 50 x 50 cm, welche eine Einlaßdüse und eine Absaugvorrichtung mit einem Membranfilter aufweist. Die Pumpe an der Absaugvorrichtung wird eingeschaltet und auf einen Durchfluß von 27 l/min eingestellt Über einen an der Einlaßdüse angebrachten Probentrichter werden 400 mg Probe durch Druckluftstoß in die Kammer eingeblasen und die Pumpe 4 Minuten später abgestellt Das Gewicht des auf dem Membranfilter abgelagerten Staubes, multipliziert mit dem empirischen Faktor 9,4, ergibt die mittlere Staubkonzentration in der Kammer.

Beispiel 1 (Vergleich)

ohne Beschichtungsmittel

Beispiel 2 (erfindungsgemäß)

+ 1,5 % Isoeikosan ( = 2,2,4,4,6,6,8,8,10-Nonamethyl-undecan)

Beispiel 3 (erfindungsgemäß)

+ 2,0 % Isoeikosan

Beispiel 4 (erfindungsgemäß)

+ 2,5 % Isoeikosan

Beispiel 5 (erfindungsgemäß)

+ 3,0% Isoeikosan

Beispiel 6 (Vergleich)

+ 2,5 % Paraffinöl flüssig mit 180 mPa.s/23 °C + 0,25 % Natriumlaurylsulfat

Prüfergebnisse

| Beispiel | Zusatz | Verarbeitungszeit | Abbindezeit u.Temperatur | Fließmaß | Druckfestigkeit 850°C | Abbinde-Expansion | thermische Expansion | Staubentwicklung |
|---|---|---|---|---|---|---|---|---|
| 1 | ohne | 3,5 Min. | 6 Min./75°C | 140 mm | 20,1 $N/mm^2$ | 0,25 % | 1,2 % | 152 $mg/m^3$ |
| 2 | + 1,5 % Isoeikosan | 3,75 Min. | 6 Min./74°C | 158 mm | 19,2 $N/mm^2$ | 0,24 % | 1,2 % | 103 $mg/m^3$ |
| 3 | + 2,0 % Isoeikosan | 3,75 Min. | 6 Min./73°C | 153 mm | 18,8 $N/mm^2$ | 0,21 % | 1,2 % | 88 $mg/m^3$ |
| 4 | + 2,5 % Isoeikosan | 3,75 Min. | 6 Min./73°C | 150 mm | 17,6 $N/mm^2$ | 0,19 % | 1,2 % | 67 $mg/m^3$ |
| 5 | + 3,0 % Isoeikosan | 4 Min. | 6,25 Min./72°C | 150 mm | 15,2 $N/mm^2$ | 0,17 % | 1,2 % | 51 $mg/m^3$ |
| 6 | + 2,5 % Paraffinöl + 0,25 % Natriumlaurylsulfat | 4 Min. | 6,5 Min./71°C | 145 mm | 14,9 $N/mm^2$ | 0,38 % | 1,0 % | 69 $mg/m^3$ |

## Patentansprüche

1. Gips- und phosphatgebundene Einbettmassen, die frei von anionischen grenzflächenaktiven Mitteln sind, dadurch gekennzeichnet daß sie Iso-Paraffine der Formel

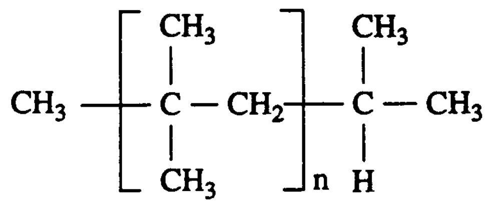

worin n 2, 3, 4 oder 5 bedeutet, enthalten.

2. Einbettmassen nach Anspruch 1, dadurch gekennzeichnet, daß sie als Iso-Paraffin 2,2,4,4,6,6,8-Heptamethyl-nonan und/oder 2,2,4,4,6,6,8,8,10-Nonamethyl-undecan enthalten.

3. Einbettmassen nach den Ansprüchen 1 und 2, dadurch gekennzeichnet, daß sie 0,5 bis 5 Gew.-% des Iso-Paraffins enthalten.

4. Einbettmassen nach den Ansprüchen 1 und 2, dadurch gekennzeichnet, daß sie 1,5 bis 3 Gew.-% des Iso-Paraffins enthalten.

5. Gipsgebundene Einbettmassen nach den Ansprüchen 1 bis 4, dadurch gekennzeichnet, daß sie Quarz, Cristobalit, Calciumsulfat-Halbhydrat und Zuschlagstoffe enthalten.

6. Phosphatgebundene Einbettmassen nach den Ansprüchen 1 bis 4, dadurch gekennzeichnet, daß sie Quarz, Cristobalit, Magnesiumoxid, Ammoniumphosphat und Zuschlagstoffe enthalten.

7. Verfahren zur Herstellung von Einbettmassen nach den Ansprüchen 1 bis 6, dadurch gekennzeichnet, daß man dem Gemisch der pulverförmigen Komponenten bzw. Teilen hiervon unter Rühren das Iso-Paraffin zusetzt.

8. Verwendung von Einbettmassen nach den Ansprüchen 1 bis 6 zur Herstellung von Gußstücken.

## Claims

1. Plaster- and phosphate-bound investment compounds which are free of anionic surface-active agents, characterized in that they contain iso-paraffins of the formula

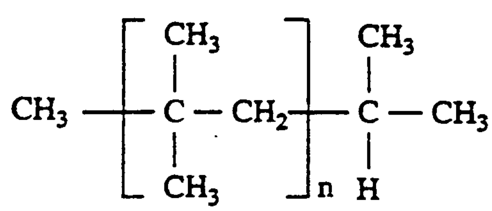

in which n denotes 2, 3, 4 or 5.

2. Investment compounds according to Claim 1, characterized in that they contain 2,2,4,4,6,6,8-heptamethyl-nonane and/or 2,2,4,4,6,6,8,8,10-nonamethyl-undecane as iso-paraffin.

3. Investment compounds according to Claims 1 and 2, characterized in that they contain 0.5 to 5% by weight of the iso-paraffin.

4. Investment compounds according to Claims 1 and 2, characterized in that they contain 1.5 to 3% by weight of the iso-paraffin.

5. Plaster-bound investment compounds according to Claims 1 to 4, characterized in that they contain quartz, cristobalite, calcium sulphate hemihydrate and additives.

6. Phosphate-bound investment compounds according to Claims 1 to 4, characterized in that they contain quartz, cristobalite, magnesium oxide, ammonium phosphate and additives.

7. Process for the preparation of investment compounds according to Claims 1 to 6, characterized in that the iso-paraffin is added to the mixture of the components or parts thereof, in the form of a powder, while stirring.

8. Use of investment compounds according to Claims 1 to 6 for preparing cast appliances.

**Revendications**

1. Revêtements liés au gypse et au phosphate, qui sont dépourvus d'agents anioniques à activité interfaciale, caractérisés en ce qu'ils contiennent des isoparaffines de formule

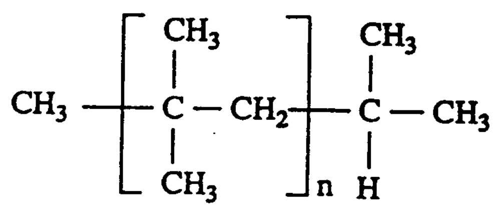

dans laquelle n a la valeur 2, 3, 4 ou 5.

2. Revêtements suivant la revendication 1, caractérisés en ce qu'ils contiennent code isoparaffine du 2,2,4,4,6,6,8-heptaméthyl-nonane et/ou du 2,2,4,4,6,6,8,8,10-nonaméthyl-undécane.

3. Revêtements suivant les revendications 1 et 2, caractérisés en ce qu'ils contiennent 0,5 à 5 % en poids de l'isoparaffine.

4. Revêtements suivant les revendications 1 et 2, caractérisés en ce qu'ils contiennent 1,5 à 3 % en poids de l'isoparaffine.

5. Revêtements liés au gypse suivant les revendications 1 à 4, caractérisés en ce qu'ils contiennent du quartz, de la cristobalite, du sulfate de calcium hémihydraté et des additifs.

6. Revêtements liés au phosphate suivant les revendications 1 à 4, caractérisés en ce qu'ils contiennent du quartz, de la cristobalite, de l'oxyde de magnésium, du phosphate d'ammonium et des additifs.

7. Procédé de production de revêtements suivant les revendications 1 à 6, caractérisé en ce qu'on ajoute l'isoparaffine au mélange des composants en poudre ou de parties de ces composants, sous agitation.

8. Utilisation de revêtements suivant les revendications 1 à 6 pour la production de pièces coulées.